(19) Europäisches Patentamt
European Patent Office
Office européen des brevets

(11) EP 4 574 206 A1

(12) EUROPEAN PATENT APPLICATION

(43) Date of publication:
25.06.2025 Bulletin 2025/26

(21) Application number: 23307374.1

(22) Date of filing: 22.12.2023

(51) International Patent Classification (IPC):
A61N 7/02 (2006.01)   A61B 8/08 (2006.01)
A61N 7/00 (2006.01)

(52) Cooperative Patent Classification (CPC):
A61N 7/02; A61B 8/5223; A61B 8/5269;
A61N 7/022; A61N 2007/0039; A61N 2007/0043;
A61N 2007/0078

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicants:
• Cardiawave
92300 Levallois-Perret (FR)
• Institut National de la Santé et de la Recherche
Médicale
75013 Paris (FR)
• Centre National de la Recherche Scientifique
75016 Paris (FR)
• Ecole Supérieure de Physique et de Chimie
Industrielles de la Ville de Paris
75005 Paris (FR)

(72) Inventors:
• PERNOT, Mathieu
75001 PARIS (FR)
• KWIECINSKI, Wojciech
75012 PARIS (FR)
• SUAREZ, Daniel
93400 SAINT-OUEN (FR)
• MAGNIER, Clara
78620 L'Etang-la-Ville (FR)

(74) Representative: Atout PI Laplace
Immeuble Up On
25 Boulevard Romain Rolland
CS 40072
75685 Paris Cedex 14 (FR)

(54) METHOD FOR DETECTING CAVITATION

(57) The invention relates to a computer-implemented method for identifying cavitation produced by an ultrasound therapy device, the ultrasound therapy device comprising at least one transducer configured for emitting ultrasound waves at a target and for receiving backscattered signal from the target, the method comprising the following steps of:

a) arranging in a raw matrix a series of sampled acquired measurements signals, each sampled acquired measurement signals representing a backscattered signal received from the target by the at least one transducer subsequently to the emission by the at least one transducer of an emitting signal configured to nucleate cavitation inside the target, the sampled acquired measurements signals being aligned in the matrix to match their respective samples to the same time acquisition after the beginning of the emitting signal.

b) determining from the matrix the presence or absence of one or more variable samples values through the series of sampled acquired measurements signals corresponding to cavitation.

Fig. 2

**Description**

The invention relates to a method for detecting cavitation, more particularly a method for detecting cavitation emitted by an ultrasound apparatus.

Acoustic cavitation is used in multiple biomedical applications to generate a mechanical (and sometimes thermal) effect in tissues. Since it has an important impact on the tissues, it is crucial to well monitor its presence and position in the patient body. Because the cavitation cloud nucleated by focused ultrasound is hyperechoic, B-mode imaging is used in several applications to observe cavitation during therapy. However, for clinical application, the quality of B-mode imaging might be greatly affected by the presence of soft tissues between the imaging probe and the cavitation cloud. Hence, it is important to incorporate a cavitation detection system to cavitation-based ultrasound therapeutic medical devices.

Passive cavitation detection (PCD) and passive cavitation imaging (PCI) described in document S. I. Madanshetty, R. A. Roy, et R. E. Apfel, J. Acoust. Soc. Am., vol. 90, no 3, p. 1515-1526, sept. 1991, are widely used methods to detect cavitation. They consist in having a transducer different than the therapeutic transducer (or a hydrophone) "listening" to the echoes of the signal emitted by the therapeutic transducer. A backscattered signal is acquired and inertial cavitation is known to have a typical broadband spectral signature. Several indicators have been calculated from PCD signals to characterize inertial cavitation: the inertial cavitation dose (the root-mean-square amplitude of the broadband noise in the frequency domain), the probability of cavitation occurrence and the cavitation persistence. The analysis of broadband noise is the most used method for the analysis of PCD and PCI signal. Also, cavitation imaging and analysis can be enhanced with the use of ultrafast ultrasound imaging since it increases the frame of acquisition, and allows a better discrimination of the signals.

However, these solutions require a second transducer in order to detect cavitation, which is a drawback for clinical applications because it increases the electronic complexity required to acquire and process the radiofrequency signals from the elements of the transducer. Furthermore, having two different transducers also means that care must be taken so that the two transducers are mechanically aligned, increasing sources of error.

It is known from R. A. Roy, S. I. Madanshetty, et R. E. Apfel, J. Acoust. Soc. Am., vol. 87, no 6, p. 2451-2458, june1990 another method called "active cavitation detection" (ACD) methods. This system also uses a different transducer than the therapeutic one to detect cavitation. In the case of ACD, the second transducer emits a signal and receives the echoes backscattered by the bubbles of the cavitation. Because this solution also uses another transducer, it presents the abovementioned drawbacks; moreover it has been suspected to have an impact on the nucleated cavitation cloud.

More recently K.-A. Saalbach, J. Twiefel, et J. Wallaschek, Ultrasonics, vol. 94, p. 401-410, april 2019 described the possibility to detect cavitation with the transducer used to nucleate the cavitation. They described the use of a transducer with a resonance frequency of 20.64 kHz, corresponding to a low frequency which is not typically used in biomedical applications. The processing of the acquired signal to detect cavitation consisted in studying the presence and amplitude of the components at $\frac{n}{2} \times f_0$ with $n$ = 3, 5, 7. However, the used resonance frequency is lower than the frequency used in biomedical applications, as typical therapy transducers have a narrow bandwidth (around 50%) around their central frequency ($f_0$) which significantly limits signal detection at $\frac{n}{2} \times f_0$ with $n$ = 3, 5, 7.

Self-sensing transducers have also been used for aberration correction in ultrasonic imaging application and for histotripsy applications. In these solutions, the same element is used to emit and receive signal. It is known from document J. R. Sukovich et al., IEEE Trans. Ultrason. Ferroelectr. Freq. Control, vol. 67, no 6, p. 1178-1191, June 2020 to use signal which is first acquired through the skull by using a pressure level configure to not nucleate cavitation in order to obtain the echoes caused by the skull; then the cavitation signal is acquired and the signal without cavitation is subtracted. Finally, the cavitation localization is determined with an algorithm.

An important limitation of this method is the need of a reference signal, which is not always possible *in vivo* because of tissue motions.

The invention intends to obviate at least partly the drawbacks of the prior art.

The invention aims to provide a method and a device to automatically detect cavitation while simplifying the instrumental detection, avoiding the alignment difficulties between two ultrasonic devices, enhancing signal detection and usable under tissue motion.

To that end, the invention relates to a computer-implemented method for identifying cavitation produced by an ultrasound therapy device, the ultrasound therapy device comprising at least one transducer configured for emitting ultrasound waves in a target and for receiving backscattered signal from the target, the method comprising the following steps of:

a) arranging in a raw matrix a series of sampled acquired measurements signals, each sampled acquired measurement signals representing a backscattered signal received from the target by the at least one transducer subsequently to the emission by the at least one transducer of an emitting signal configured to nucleate cavitation inside the target, the sampled acquired measurements signals being aligned in the matrix to match their respective samples to the same time acquisition after the beginning of the emitting signal,

b) determining from the matrix the presence or absence of one or more variable samples values along the series of sampled acquired measurements signals corresponding to cavitation.

Hence, the method of the invention is not carried out on a single measure received from the target but uses a series of measurements allowing a better determination of the variability of the high energy values through the series. Indeed, the inventors found out that the backscattered signal from the emitting signal show stable/constant amplitude and positions through the different measurements signals while the cavitation, because of its erratic apparitions, shows variable and unstable amplitudes through a range of positions on the different measurements signals. This allows detecting sample values in the series of measurement corresponding to cavitation.

The method of the invention can be advantageously carried out on a moving target, especially on a target with a pattern movement, like the heart of a patient, and on target impacted by breathing movement like the liver of a patient.

Besides, analysing at which time on the acquired signal the cavitation appears allows obtaining the location of the latter.

In the invention, the ultrasound device configured for nucleating cavitation in the target may emit focused waves at a focal zone of the target or may emit diverging waves.

• STEP A)

Step a) corresponds to the arrangement in a raw matrix of a series of sampled acquired measurements signals from the target. The raw matrix corresponds then to a two dimensional temporal representation of the series of measurements, wherein one of the dimensions represents the recording time of each backscattered signal and the other dimension represents the start time of each measurements signal.

An acquired measurement signal corresponds to the voltage amplitude values recorded from the backscattered signal from the beginning of one pulse of emitting signal to at most the moment before the following pulse. Hence, each sample corresponds to an amplitude value of one sampled acquired measurement signal at a time of sampling.

Especially, each acquired signal lasts for a minimal time which can be determined using the following formula:

$$T_{Si} = \frac{2 \times D}{S}$$

wherein $T_{Si}$ is the minimal time length of an acquired signal,

D is the distance between the at least one transducer and the focal zone, and

S is the mean speed of the ultrasound wave on distance D,

In case the target is a body part, the mean speed S can be considered as 1540 m/s.

Each signal can be shorter than the time between two pulses of emitting signal.

Two pulses of emitting signal may be separated by a minimal time length which can be determined using the following formula:

$$T_{Se} = \frac{2 \times D}{S} + T_{ED}$$

wherein $T_{Se}$ is the minimal time length separating two emitting signal,

D is the distance between the at least one transducer and the focal zone,

S is the mean speed of the ultrasound wave on distance D, and

$T_{ED}$ is the time of emission duration.

The series of measure may comprise at least 20 acquired measurements signals, notably at least 50, especially at least 100, for example 200.

Each sampled acquired measurement signal comprises at least 20 samples, notably at least 40 samples.

Each acquired measurement signal are sampled and stored in a matrix.

The whole sampled signals may be stored along the rows or along the columns of the matrix.

Hence each sample corresponds to a particular amplitude value of the signal obtained at a time of sampling.

The sampling frequency may be from 5 to 15 times the at least one transducer frequency, for example from 3MHz to 15MHz.

To be able to compare the samples values through the different sampled signals of the series, the sampled signals are aligned along the matrix (rows or columns). Hence each row, or each column, contains the samples of each sampled signal of the same time of recording after the beginning of the emitting signal.

In case the sampled signals of the series do not comprise the same amount of samples, they may be modified to match the exact same amount of samples. To that end, they may be aligned by matching the time of record of their samples and the sampled values which do not have a time of record shared by all sampled signal may be adapted. In particular, according to a first adaptation the sample values of the sampled signals beyond (before or after) those of the shortest sampled signal may be cut. Alternatively, according to a second adaptation, the sampled signals shorten than the longest sampled signal may be completed with corresponding sampled values equal to 0.

• **STEP B)**

Step b) of the invention allows determining the presence or absence of one or more samples corresponding to cavitation in the raw matrix.

Each sampled signal can be segmented into different time windows, each corresponding to at least one sample, namely an emission window, at least one echo window and possibly a cavitation window.

The time windows are considered along the columns of the raw matrix when the sampled signals are aligned along the rows and *vice-versa.*

The emission window corresponds to amplitude values emitted by the emitting signal. Indeed, because the signal is acquired with the same transducer that also emits the emitting signal, the beginning of an acquired signal does not correspond to backscattered signal but directly to amplitude values of the emitting signal. The emission window then lasts for the duration of the pulse of the emitting signal. Because the duration of the pulse is known, the samples within the emission window may be directly forced to be zero in this step. Otherwise, the samples within the emission window may be filtered, as described in details below.

An echo window comprises samples corresponding to amplitude values emitted solely by the echoes of the emitting signal from the target. For the purpose of the invention, the echo window does not comprise cavitation, but a cavitation window may comprise echoes of the emitting signal, as described below. The echo window does not overlap the emission window. The echo windows comprise samples which values are considered stable and repeatable through the different acquired signal measurements, notably the value of the samples may differ from 20 to 75% through the different sampled signals of the echo windows. Such a difference is greatly less than the difference of the values of the samples in the cavitation windows, which ranges between 200% and 600%. The echo windows comprise high sample values, notably sample values between 15% and 200% of the cavitation maximal amplitude expected possible range.

Different methods can be applied to determine the echo window(s) in each sample signal.

In one embodiment, the echo window position in each acquired sampled signal may be determined in a preliminary step on a series of measures acquired in the same operation conditions, and applied to the series of measure of step a).

In another embodiment, the echo window may be determined on the raw matrix or on a filtered matrix obtained at substep i) of step b) and described in details below as a time window between the emission window and the time of a determined beginning of focalisation at the focal zone of the emitting signal.

The beginning of focalisation at the focal zone of the emitting signal may be determined by the following formula:

$$B_{F=} \frac{2 \times D_S}{S}$$

wherein $B_F$ corresponds to the time of the beginning of focalisation,

$D_S$ corresponds to the lowest steering distance achievable by the transducer (corresponding to the back and forth movement of the wave), and

S is the mean speed of the ultrasound wave on distance $D_S$.

In another embodiment, the echo window may be determined on a filtered matrix obtained at substep i) of step b) and described in details below, as a time window after the emission window and outside of a zone beginning two times the emitting signal duration before the maximum sample value of each sampled signal and ending two times the emitting signal duration after the said maximal value. In this aspect of the invention, the maximum sample value of each sampled signal is assumed to correspond to cavitation. Because the signal of cavitation can be considered shorter than four times the emitting duration, each sample value which is outside of a time window of four times the emitting duration around cavitation can be considered to be in an echo window.

Finally, a sample signal may comprise a cavitation window comprising sample values generated by cavitation at the focal zone of the target. The purpose of step b) is to identify the presence of such sample values. In contrast to the echo windows, the cavitation window varies more in position and amplitude values through the different sampled signals. The cavitation window may also comprise sample values pertaining to the echoes of the emitting signal, which will be differentiated from the sample(s) corresponding to cavitation, as described in details below.

Step b) may be at least partially carried out by an artificial intelligence engine.

The artificial intelligence engine can be based on any model of artificial intelligence (AI). As artificial intelligence models, for example AI based classifiers may be used, machine learning models may be used, e.g. deep learning-based models, neural networks, logistic regression models, random forest models, support vector machine models or tree-based models with decision trees as a basis may be used. The AI is especially a convolution neural network pertaining to the deep learning-based models.

In one embodiment, the artificial intelligence engine carries out the entire step b). In this case the artificial intelligence engine may be trained with a pool of series of measurements each arranged according to the raw matrix of step a), and each associated with an annotation of the presence or absence of cavitation in the series. In this aspect of the invention, the artificial intelligence engine has a data structure containing information reflecting relationship between sample values of the raw matrix and the presence of cavitation. Hence, from the raw matrix provided at step a), the artificial intelligence engine returns the determination of presence or absence of sample(s) corresponding to cavitation in the series of sampled signals.

In one embodiment, step b) can be carried out by:

i) obtaining a filtered matrix by applying a filter configured for identifying and treating the at least one sample of the raw matrix corresponding to at least one echo of the emitting signal returned by the target by removing or lowering its value, and obtaining filtered samples, and

ii) determining from the filtered matrix the presence or absence of amplitudes values corresponding to cavitation.

In substep i) of step b), sample values corresponding to the emitting signal are also filtered in case they were not previously put to 0.

One or both of these sub-steps may be carried out by an artificial intelligence engine.

Especially, for step i), the artificial intelligence engine may be trained on a pool of raw matrix according to step a) associated with an annotation of the position of the echoes of the emitting signal, and optionally of the emitting signal itself. In this aspect of the invention, the artificial intelligence engine has a data structure containing information reflecting relationship between sample values of the raw matrix and the presence of echoes from the emitting signal and optionally of the emitting signal itself. Hence, from the raw matrix provided at step a), the artificial intelligence engine returns a filtered matrix where the samples corresponding to the echoes of the emitting signal and optionally of the emitting signal itself are treated. The treatment may be a lowering of values, a transformation to 0 or a removing of the sample(s) corresponding to the echo window(s) along the series.

Substep i) of step b) may also be carried out by applying a 2D temporal filter to the raw matrix for obtaining a filtered matrix.

In one embodiment, the 2D temporal filter applied to the raw matrix may be configured for distinguishing samples corresponding to stable values to other along the series. In particular, the filter applied to the matrix may be configured to separate the stable samples with a high coherence in the 2D temporal dimensions of the raw matrix from the unstable samples with a low 2D temporal coherence.

Especially, the 2D temporal filter applied to the matrix carries out a singular value decomposition filtering of the matrix. In the filtered matrix, the singular values are ranked from the highest to the lowest value. The obtained first singular values correspond to the most coherent signal, i.e. to echoes (and pulses if not previously filtered). The last singular values correspond to noise. The in-between singular values may correspond to cavitation. Then the first and last singular values are removed and the remaining intermediate values are kept for the continuation. The first of the intermediate singular values may correspond to the $2^{nd}$ to the $15^{th}$ singular value, while the last of the intermediate singular values may corresponds to at least the 20th value.

In one embodiment of the invention, the singular value decomposition filtering applied during substep i) of step b) may be followed by a high-pass filter, to attenuate the coherent signals corresponding to echoes remaining after the singular values decomposition filtering. In particular, the cut-off value of the high-pass filter may be the emitting signal pulse-repetition frequency divided by 3 to 5 (typically between 20Hz and 160Hz) and the attenuation slope may be from -20dB/decades to -40dB/decades.

Determination whether or not samples of filtered samples of the filtered matrix correspond to cavitation is carried out in substep ii).

As above mentioned, substep ii) may be carried out by an artificial intelligence engine. This artificial intelligence engine may be trained on a pool of filtered matrix associated with an annotation of the presence or absence of cavitation. In this aspect of the invention, the artificial intelligence engine has a data structure containing information reflecting relationship between amplitudes values of the filtered matrix and the presence of cavitation. Hence, from the filtered matrix, the artificial intelligence engine returns the determination of presence or absence of cavitation in the series of measures.

Substep ii) may also be carried out by using one more indicators.

In one embodiment, a signal-to-noise ratio indicator may be determined for each filtered sample signal as the maximal value of the filtered samples divided by the standard deviation of the filtered sample values in one echo window. If the result is higher than a predetermined threshold, the raw matrix is considered as presenting cavitation.

Alternatively or in a complementary way, an amplitude indicator may be determined as the mean of the maximal filtered sample value of each filtered sampled signal. If this mean is higher than a certain a predetermined threshold, matrix is considered as presenting cavitation.

Alternatively or in a complementary way, an energy indicator may be determined for each filtered sampled signal as the square root of the integral of the filtered samples values. Again, if the result is higher than a predetermined threshold, the filtered sampled signal, and thus the corresponding sample signal, is considered as presenting cavitation.

The threshold of each mentioned indicator is dependent from the parameters of the used at least one transducer, and may be determined in a preliminary step with acquisitions of series of measurements in water and/or with *in-vitro* set-up and/or with *in vivo* set-up of one group wherein cavitation is not nucleated and a second group wherein cavitation is nucleated. The obtained matrix can be filtered as above detailed. In particular, from this preliminary step, the threshold can be considered as the mean of a maximal value for the first group after filtering and a minimal sample value obtained in the second group after filtering. The minimal value can be considered as the highest value between the lowest sample value and the first quartile value minus 1.5 times the interquartile range. The maximal value can be considered as the lowest value between the highest sample value and the third quartile value plus 1.5 times the interquartile range.

Before step a), the method of the invention may comprise a preliminary acquisition step of carrying out a series of measures, each measure comprising acquiring the echoes received from the target by the at least one transducer as a measurement signal. In this case, all steps from the preliminary acquisition step to step b) or solely step a) and b) are performed by a computer.

In particular, the preliminary acquisition step may comprise the following sub-steps:

i) emitting by the at least one transducer an emitting signal configured to nucleate cavitation inside at the focal zone of the target, and

ii) acquiring the backscattered signal received from the target by the at least one transducer as a measurement signal.

The emitting signal may be a sinusoidal signal at a frequency ranging from 100kHz to 10MHz, and even more preferably from 500kHz to 2.5MHz, with high intensity pulsed emission form 1 to 20$\mu$s, delivered at a pulse repetition frequency ranging from 1Hz to 1000Hz, and even more preferably from 50Hz to 500Hz.

Each acquired signal is then sampled before step a).

The method may also comprise a step c) of repeating acquisition preliminary step to step b). This aspect of the invention allows monitoring the presence of cavitation in the focus zone along the therapy treatment.

The invention also relates to a computer system configured to be in communication with an ultrasound therapy device, the ultrasound therapy device comprising: at least one transducer configured for emitting ultrasound waves at a target and for receiving backscattered signal from a target, the computer system comprising one or more processors, a memory and one or more programs, wherein the one or more programs are stored in the memory and configured to be executed by the one or more processors, the one or more programs including instructions for performing, when executed by the computer system, a method for identifying cavitation as above defined. In particular, the at least one transducer are also configured for emitting ultrasound waves at a target.

Notably, the ultrasound therapy device may comprise a communication member to send the backscattered signal from the target to the computer system and receive order from the computer system.

Besides, the computer system comprising may also comprise a communication member to receive information from the ultrasound therapy device and to send orders to the ultrasound therapy device.

In one embodiment, the one or more programs include instructions for performing a preliminary acquisition step upstream step a) of carrying out a series of measures, each measure comprising the following sub-steps:

- emitting by the at least one transducer an emitting signal configured to nucleate cavitation at the focal zone of the target, and

- acquiring the backscattered signal received from the target by the at least one transducer as a measurement signal.

The therapy device of the invention uses the same at least one transducer to carry out both

- the emission of an ultrasound signal, and
- the reception of the backscattered signal from the target.

This aspect of the invention avoids double focus problems, and reduces the volume of the therapy device.

Notably, in case the therapy device comprises several transducers, it is possible to use solely one of them, or several or all of them, to record the different backscattered signal of the series of measurements.

For the emission of the signal, the therapy device may comprise a signal generator followed by a high power amplifier linked to the at least one transducer.

The transducers that can be used to emit the emitting signal can be of any type. Especially, the at least one transducer may be an array of concentric annular ultrasound transducer as described in detail in WO2016156989.

The invention also relates to a computer program product, stored on a non-transitory computer-readable data-storage medium comprising computer-executable instructions to cause a computer system to carry out a method for identifying cavitation as above defined.

The invention finally relates to a non-transitory computer-readable data-storage medium containing computer-executable instructions to cause a computer system to carry out a method for identifying cavitation as above defined.

The invention also relates to an apparatus comprising the computer system as above defined and an ultrasound therapy device configured for detection of cavitation, the ultrasound therapy device comprising: at least one transducer configured for emitting ultrasound waves at a target and for receiving backscattered signal from the target. In particular, the at least one transducer are also configured for emitting ultrasound waves at a target.

The features of the ultrasound therapy device above recited in relation with the apparatus apply *mutatis mutandis* to this aspect of the invention.

For the reception of the backscattered signal, the apparatus may comprise a high-voltage probe linked the at least one transducer and followed by an oscilloscope. The high-voltage probe allows respecting the amplitude range of the oscilloscope.

## LEGEND TO THE FIGURES

**Figure 1** represents an apparatus of the invention comprising a computer system and an ultrasound therapy device.

**Figure 2** is an organigram representing the computer-implemented method for identifying cavitation produced by an ultrasound therapy device.

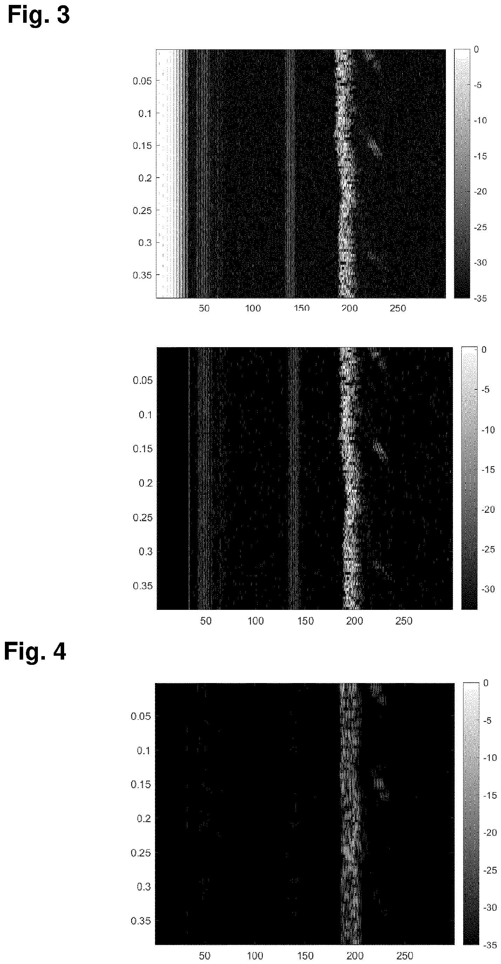

**Figure 3** represents two matrices of sampled acquired signals on a static target after emission of series of signals configured to nucleate cavitation in the target by a multi-element ultrasound transducer. The top matrix represents the raw matrix wherein each row corresponds to a sampled acquired signal between two pulses of the multi-element ultrasound transducer. X-axis is in microseconds and y-axis is in seconds. The grey scale represents the voltage amplitude in decibels of the sampled signals. The first high values represent the voltage amplitude resulting from the emitting signals. The regular lines along the columns represent echoes of the emitting signals. The crackled high intensity zone along the columns around 190 microseconds represents cavitation. On the bottom matrix, the first high values were put to 0, consequently highlighting the cavitation and the echoes.

**Figure 4** represents the top matrix of Figure 3 after application of a singular decomposition value filter. On the represented filtered matrix, the regular lines corresponding to the echoes of the emitting signal almost disappeared while the crackled intensity zone corresponding to the cavitation is still well visible.

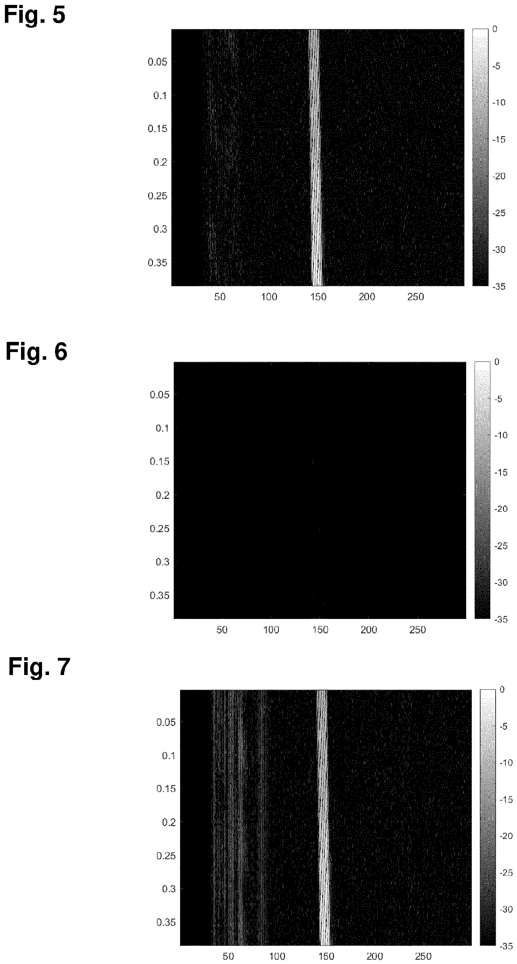

**Figure 5** represents a matrix of sampled acquired signals on a moving target after emission of series of signals not configured to nucleate cavitation in the target by a multi-element ultrasound transducer. Each row corresponds to a sampled acquired signal between two pulses of the multi-element ultrasound transducer. X-axis is in microseconds and y-axis is in seconds. The grey scale represents the voltage amplitude in decibels of sampled signals. The first high values corresponding to the voltage amplitude resulting from the emitting signals were put to 0. The regular pattern zone around 150 microseconds represents echoes of the emitting signals.

**Figure 6** represents the matrix of Figure 5 after application of a singular decomposition value filter. On the represented filtered matrix, the echoes have disappeared, and no high intensity value remains corresponding to the absence of cavitation in the raw matrix.

**Figure 7** represents the matrix of Figure 5 after application of a filter using a reference signal calculated as the mean of all pulses acquired without cavitation. On the represented filtered matrix, the echoes have not disappeared.

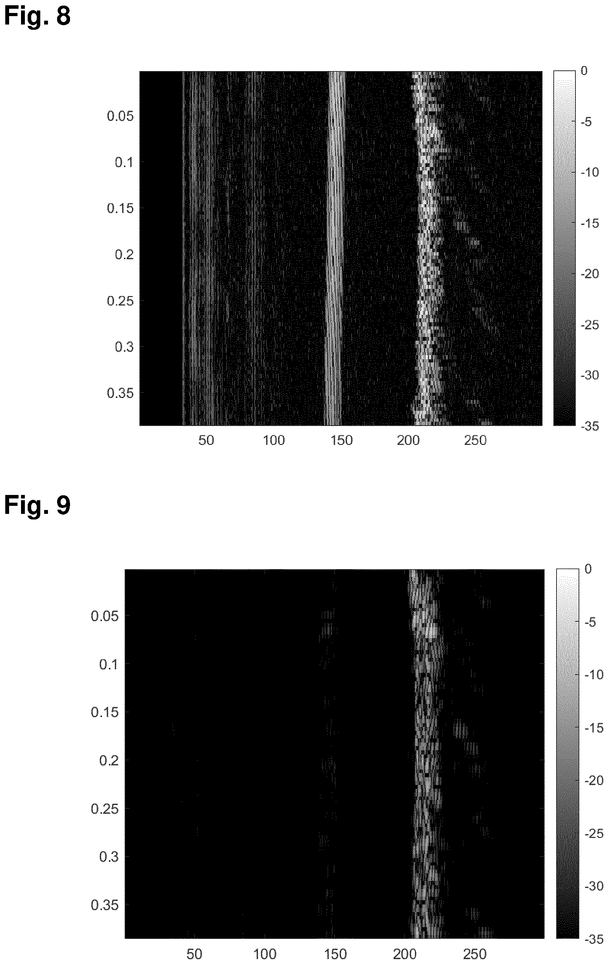

**Figure 8** represents a matrix of sampled acquired signals on a moving target after emission of series of signals configured to nucleate cavitation in the target by a multi-element ultrasound transducer. Each row corresponds to a sampled acquired signal between two pulses of the multi-element ultrasound transducer. X-axis is in microseconds and y-axis is in seconds. The grey scale represents the voltage amplitude in decibels of sampled signals. The first high values corresponding to the voltage amplitude resulting from the emitting signals were put to 0. The regular pattern zone just before 150 microseconds represents echoes of the emitting signals. The crackled high intensity zone along the columns around 210 microseconds represents cavitation.

**Figure 9** represents the matrix of Figure 8 after application of a singular decomposition value filter. On the represented filtered matrix, the echoes have disappeared, while the crackled intensity zone corresponding to the cavitation is still well visible.

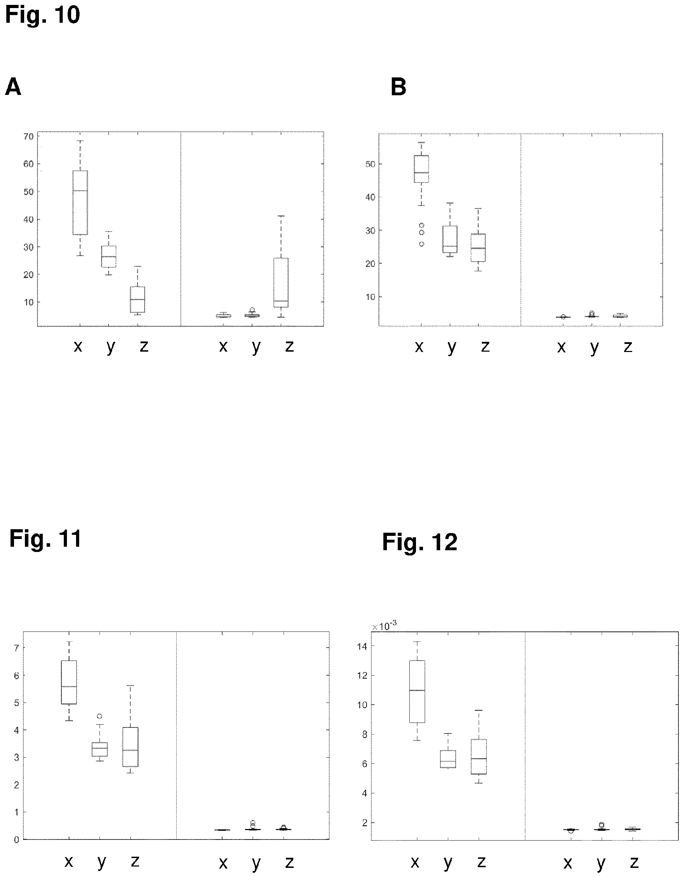

**Figure 10** represents different boxplots of a signal-to-noise (SNR) indicator values after filtering a matrix of sampled acquired signals on different static *in vitro* targets after emission of series of signals in the target by a multi-element ultrasound transducer in different situation. In **Figures 10A** and **10B,** the left insert represents a condition where the emitting signal was configured to nucleate cavitation in the target, while the right insert represent a condition where the emitting signal was not configured to nucleate cavitation in the target. In **Figure 10A,** the filter used was a comparative filter using a reference signal calculated as the mean of all pulses acquired without cavitation. In **Figure 10B,** the filter used was singular value decomposition. On both Figures 10A and 10B, x represents the water condition, y represents the static phantom condition, z represents the phantom moving condition and circles represent aberrant values above 1.5 times the interquartile range.

**Figure 11** represents different boxplots in the same condition as Figure 10B but for an amplitude indicator. x represents the water condition, y represents the static phantom condition, z represents the phantom moving condition.

**Figure 12** represents different boxplots in the same condition as Figure 10B but for an energy indicator. x represents the water condition, y represents the static phantom condition, z represents the phantom moving condition

**Figure 13** represents different boxplots in the same condition as Figure 10 but for an *in vivo* target. **Figure 13A** is the comparative experiment using filter using a reference signal calculated as the mean of all pulses acquired without cavitation and **Figure 13B** the invention using singular value decomposition as a filter.

**Figure 14** represents different boxplots in the same condition as Figure 13B but for an amplitude indicator.

**Figure 15** represents different boxplots in the same condition as Figure 13B but for an energy indicator.

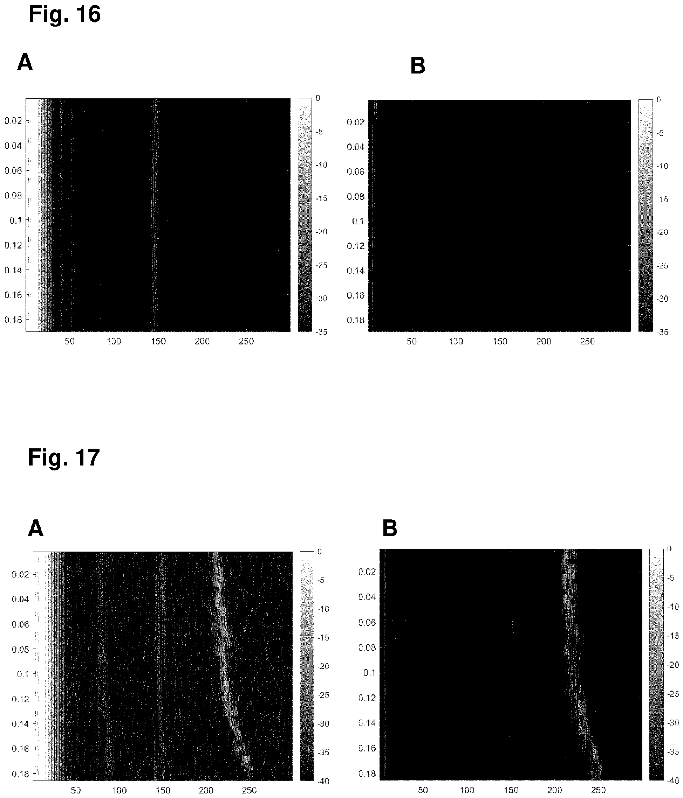

**Figure 16** represents two type of inputs for a Convolution Neural Network (CNN), wherein the inputs do not comprise sample corresponding to cavitation. **Figure 16A** represents a matrix of sampled acquired signals on a moving target after emission of series of signals configured not to nucleate cavitation in the target by a multi-element ultrasound transducer. Each row corresponds to a sampled acquired signal between two pulses of the multi-element ultrasound transducer. X-axis is in microseconds and y-axis is in seconds. The grey scale represents the voltage amplitude of sampled signals. The regular pattern zone just before 150 microseconds represents echoes of the emitting signals. It is an input for the CNN that classifies raw matrix. **Figure 16B** represents Figure 16A after application of a singular decomposition value filter. On the represented filtered matrix, the echoes have disappeared. It is an input for the CNN that classifies filtered matrix.

**Figure 17** represents two types of inputs for a CNN, wherein the inputs comprise samples corresponding to cavitation. **Figure 17A** represents a matrix of sampled acquired signals on a moving target after emission of series of signals configured to nucleate cavitation in the target by a multi-element ultrasound transducer. Each row corresponds to a sampled acquired signal between two pulses of the multi-element ultrasound transducer. X-axis is in microseconds and y-axis is in seconds. The grey scale represents the voltage amplitude of sampled signals. The regular pattern zone just before 150 microseconds represents echoes of the emitting signals. The crackled high intensity zone along the columns from 220 to 250 microseconds represents cavitation. It is an input for the CNN that classifies raw matrix.
**Figure 17B** represents Figure 17A after application of a singular decomposition value filter. On the represented filtered matrix, the echoes have disappeared, while the crackled intensity zone corresponding to the cavitation is still well visible. It is an input for the CNN that classifies filtered matrix.

## DETAILED DESCRIPTION

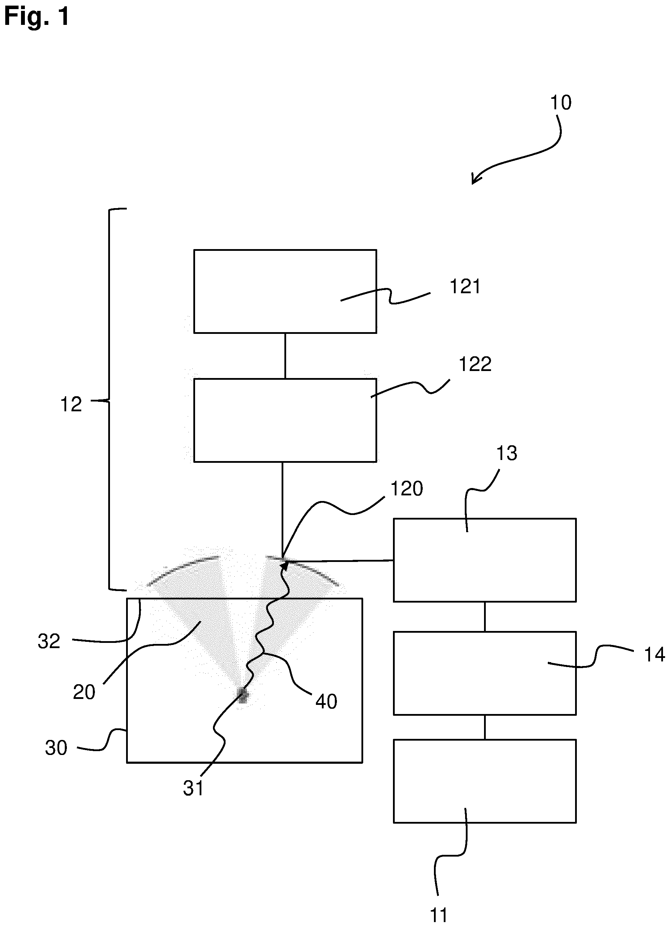

As represented in Figure 1, the apparatus (10) of the invention comprises a computer system (11) and an ultrasound therapy device (12).

The ultrasound therapy device (12) comprises at least one transducer (120) configured for on one hand emitting ultrasound waves (20) at a target (30) *via* its surface (32) adapted for nucleating cavitation within the target (30) and for on another hand receiving a backscattered signal (40) from the target (30). Notably, the at least one transducer (120) are configured for emitting focused ultrasound waves (20) at a focal zone (31) of a target (30) as represented. In particular, the ultrasound therapy device (12) comprises an array of transducers (120) for emitting ultrasound waves (20) and at least one of the said transducers (120) is configured for receiving the backscattered signal (40).

For generating the emitting ultrasound waves (20), the therapy device (12) may comprise a signal generator (121) followed by a high power amplifier (122) linked to the at least one transducer (120).

The ultrasound therapy device (12) may further comprise a communication member (not shown) to send information to the computer system (11) and notably the different signals of the backscattered signal (40) acquired by the at least one transducer (120), and to receive order(s) from the computer system (11). The communication member may be wireless or wired.

Alternatively or in a complementary way, the apparatus (10) may comprise, between the ultrasound therapy device (12) and the computer system (11), a high-voltage probe (13) linked to the at least one transducer (120) and followed by an oscilloscope (14). The high-voltage probe (13) allows respecting the amplitude range of the oscilloscope (14). The oscilloscope transmits the signals to the computer system (11), where the signals are stored and sampled. Such an oscilloscope (14) allows monitoring the well reception of the echoes signals and their aspect.

The computer system (11) comprises one or more processors, a memory notably for storing the echoes signals and one or more programs, and notably a communication member to receive information from the ultrasound therapy device (12) and send order to the latter. The communication member may be wireless or wired.

The one or more programs are configured to be executed by the one or more processors. The one or more programs comprise instructions for performing, when executed by the computer system (11), a method for identifying cavitation from the echoes signal (40).

The one or more programs may further comprise instructions to be executed by the ultrasound therapy device (12). In particular, the one or more programs may further comprise instructions to pilot series of emitting signal by the ultrasound therapy device (12).

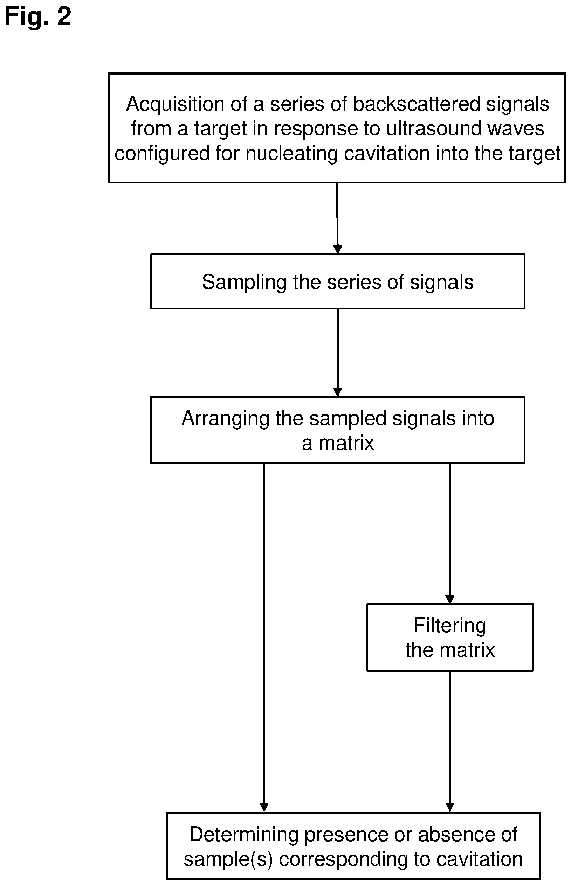

Figure 2 represents an organigram of the computer-implemented method for identifying cavitation produced by an ultrasound therapy device (12). The first and optional represented step corresponds to the acquisition of a series of echoes signals in response to ultrasound waves emitted by the ultrasound therapy device (12). Then the series is sampled and arranged in a matrix along the rows or the columns. From the matrix, the presence or absence of one or more samples corresponding to cavitation can be directly determined (notably *via* an intelligence engine), or the matrix may be filtered before to highlight difference between sample(s) corresponding to echoes of the emitting signal and sample(s) corresponding to the cavitation.

## EXAMPLES

### Material

The inventors tested the detection of cavitation *in vitro* in

- water alone,

- water surmounted by a fixed phantom tissue made of Zerdine® hydrogel (CIRS Incorporation, USA) phantom with a 6cm thickness and
- water surmounted by a moving phantom tissue (corresponding to the same Zerdine® phantom with a back-and-forth movement of 8mm in the ultrasound axis of propagation with a speed of 7mm/s to mimic the heart movement),

as well as *in vivo* where acquisitions were made on two swine by positioning the therapy transducer on their chest and by targeting the aortic valve.

Focused ultrasound waves were emitted *in vitro* and *in vivo* with a 1.1 MHz focused mono-element transducer ($f$ = 150mm, $f/D$ = 0.93; H-301, Sonic Concepts, USA), called hereafter "therapy transducer". This transducer was driven with a 2.5kW power amplifier (RITEC, USA). The therapy transducer was used to emit 12 cycles pulse, 12 $\mu$s long, delivered at a pulse repetition frequency of 100Hz.

A returned signal from the phantom tissue was acquired at 12.5MHz between each pulse by one of the elements of the therapy transducer connected to an oscilloscope (MDO4054B-6, Tektronix®, USA) and a computer on which is installed the software Matlab® (Mathworks®) for the acquisitions, filtering and indicators calculation and a Python® integrated development environment with Tensorflow® and Keras® libraries for AI applications.

Acquisitions lasted 0.4s each which corresponds to a series of 40 acquisitions at a pulse-repetition frequency of 100Hz.

The signals were sampled at a frequency of 12.5MHz.

**Example 1: Proof of concept**

The inventors first assayed the detection of cavitation on the fixed phantom tissue.

Focused ultrasound waves with the therapy transducer were emitted below the phantom tissue in the water environment. The different acquired signals were sampled and arranged in a matrix represented in Figure 3, wherein each row represents an acquired signal. Because cavitation was expected to appear before 300 $\mu$s after the beginning of the pulse, signal after this duration was removed from the matrix. On the top representation, the first high value echoes corresponding to the pulse of emitting signal is visible. On the bottom representation, these high values were forced to be equal to 0, in order to better visualise the different echoes of the emitted signal (around 50$\mu$s and between 130$\mu$s and 150$\mu$s) and the cavitation (around 200 $\mu$s).

As observed in Figure 3, the amplitude and position of the echoes from the emitted signal are repeatable through the different pulses, while the amplitude and position of the cavitation vary from one pulse to another.

Then, a 2D temporal filter as a singular value decomposition is applied to the matrix wherein singular values between the 4th and 30th were kept for the continuation, and the filtered matrix is presented in Figure 4. As it can be noticed the echoes around 50$\mu$s and between 130$\mu$s and 150$\mu$s have been removed. Only the crackled signal around 200$\mu$s corresponding to cavitation remains. Therefore, the method was demonstrating as allowing isolating the amplitude level from the raw matrix corresponding to solely the cavitation.

**Example 2: Pulses' echoes removing on a moving object**

The inventors then assayed removing the pulse's echoes on the moving phantom tissue.

First, the power of the therapy transducer was here reduced to 570W to not nucleate cavitation below the moving phantom tissue in the water environment.

The different acquired signals were sampled and arranged in a matrix represented in Figure 5. In a similar way as for Example 1, the first high amplitude values corresponding to the pulse were put to 0 and the signal after 300 $\mu$s was removed from the matrix. As shown, and contrary to the matrix of Figure 3, the echoes (around 50 and 150 $\mu$s), are different from one acquired signal to another.

Nevertheless, when a 2D temporal filter carrying out singular value decomposition (SVD) is applied on the matrix wherein singular values between the 4th and 30th were kept for the continuation, the resulting filtered matrix, represented in Figure 6, does not show any high amplitude value around 50 and 150 $\mu$s. No high intensity value remains corresponding to the absence of cavitation in the raw matrix.

On another hand, when using, as a comparative filter from the prior art, a filter using a reference signal calculated as the mean of all pulses acquired without cavitation, the amplitude of the echoes remains comparatively high, as shown in Figure 7.

Then, the power of the therapy transducer was here increased to 1847W to nucleate cavitation below the moving phantom tissue.

The different acquired signals were sampled and arranged in a matrix represented in Figure 8. In a similar way as for Example 1, the first high amplitude values corresponding to the pulse were put to 0 and the signal after 300 $\mu$s was removed from the matrix. As the matrix of Figure 5, the echoes (around 50 and 150 $\mu$s) are different from one acquired signal to another. Cavitation signal is after 200$\mu$s and as observed in Figure 3, the amplitude and position of the echoes from the emitted signal are repeatable through the different pulses, while the amplitude and position of the cavitation vary from one pulse to another.

Nevertheless, when a 2D temporal filter carrying out singular value decomposition (SVD) is applied on the matrix, the resulting filtered matrix, represented in Figure 9, does not show any high amplitude value around 50 and 150 $\mu$s. High intensity value remains at 200$\mu$s corresponding to the cavitation in the raw matrix.

This demonstrates that singular value decomposition can efficiently filters the matrix as well as on static target than a moving target without filtering cavitation signal, contrasting with the filter used on the prior art which is inefficient on moving target.

## Example 3: Automated cavitation identification

The inventors then assayed on different targets: water, fixed phantom tissue and moving phantom tissue to automatically identify cavitation.

The conditions of Example 1 were repeated on the different targets, in cavitation and no cavitation conditions.

The obtained matrices were filtered in a first case thanks to singular value decomposition wherein singular values between the 4th and 30th were kept for the continuation and further filtered in a high-pass filter with a cut-off value of 20Hz and an attenuation slope of - 20dB/decades, and in a second comparative case with the comparative filter described in Example 2.

From the filtered matrix, identification of high singular values as cavitation were carried out using a signal-to-noise ratio (SNR) indicator calculated as the maximal value of a row divided by the standard deviation of the echo window defined as all sample values outside of a window of 24$\mu$s before the maximum sample value of each sample signal and 24$\mu$s after this maximum.

The value of the SNR of each acquired signal in the different conditions is represented on Figure 10 as box plots (A: comparative; B: invention). As clearly shown, the comparative filter does not allow differentiating cavitation and no cavitation conditions on water and moving phantom targets, while with singular value decomposition high amplitudes from cavitation are significantly detected. Threshold for cavitation with the used transducer can be easily determined from those representations using the method of the invention.

The same experiment was repeated but with amplitude indicator (Figure 11) calculated as the mean of maximal amplitude of each row of the filtered matrix, and an energy indicator (Figure 12) calculated as the mean of the square root of the integral of each squared row. In both cases, the results are similar to the ones obtained with SNR indicator.

The experiments were then repeated *in vivo.*

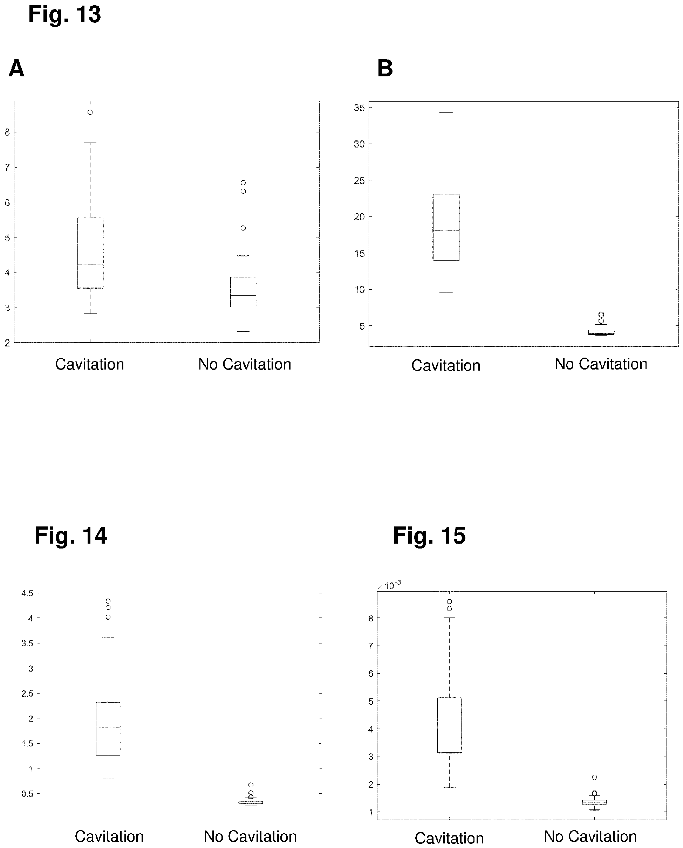

The results are presented on Figures 13 to 15 and are similar to the preceding ones. These experiments demonstrate the efficiency of the SVD and high-pass filter combined with different indicator in the automated identification of cavitation *in vitro* as well as *in vivo.*

Then accuracy of the automatic detection was calculated as the number of correct predictions on the total number of predictions. Accuracy of 1 was obtained for all *in-vitro* medium and all metrics which correspond to classifications without errors. For *in-vivo* acquisitions, the accuracy of the SNR was 1, while the accuracy of the amplitudes and power were 0.99 which is still a very good classification. Overall, this demonstrates that the SNR is more robust than the amplitude and the power, but that all these indicators can be used for automatic detection.

## Example 4: Artificial intelligence

The inventors then assayed to determine the presence and absence of cavitation using artificial intelligences.

Conventional neural networks (CNN) developed in python with Tensorflow® and Keras® libraries were trained with a pool of series of measurements each arranged according to the raw matrix of step a) of the method of the invention, each series being associated with an annotation of the presence or absence of cavitation. One of the conventional neural networks ($CNN_a$) was then selected as the most relevant CNN on testing series of measurements.

Besides, conventional neural networks developed in python with Tensorflow® and Keras® libraries were trained with a pool of series of measurements each arranged according to the raw matrix of step a), filtered thanks to singular value decomposition combined with a high-pass filter and each associated with an annotation of the presence or absence of cavitation in the series. One of the conventional neural networks ($CNN_b$) was then selected as the most relevant CNN on testing series of measurements.

Matrix inputs for $CNN_a$ and $CNN_b$ for the experiment are presented in Figures 16 and 17.

For Figure 16, the power of the therapy transducer was here reduced to 934W to not nucleate cavitation below the moving phantom tissue.

For Figure 17, the power of the therapy transducer was increased to 1054W to nucleate cavitation below the moving phantom tissue.

The matrix represented in Figures 16A and 17A were given as inputs of the convolutional neural network $CNN_a$. Of course, the series of measurements was not part of the training or testing set that were used to develop the neural network.

The matrix represented in Figures 16B and 17B were given as inputs of the convolutional neural network $CNN_b$. Again, the series of measurements was not part of the training or testing set that were used to develop the neural network.

Besides, the conditions of Example 2 were used to determine the signal-to-noise ratio (SNR) of the acquisitions represented on Figures 16B and 17B, used as positive control.

The values of the corresponding SNR and the answers of the two neural networks are displayed on the below table:

| Acquisition | SNR | $CNN_a$ | $CNN_b$ |
|---|---|---|---|
| Figure 16: no cavitation | 4,4 | No cavitation | No cavitation |
| Figure 17: cavitation | 22,4 | Cavitation | Cavitation |

The SNR obtained for Figure 16B was below a threshold of 7.14 determined with acquisitions of Example 3. This confirms the relevance of the SNR indicator for determining absence of cavitation. Besides, both convolution neural networks $CNN_a$ and $CNN_b$ also successfully determine the absence of cavitation.

The SNR obtained for Figure 17B was above the threshold (7.14). Again, this confirms the relevance of the SNR indicator for determining the presence of cavitation. Besides, both convolution neural networks $CNN_a$ and $CNN_b$ also successfully determine the presence of cavitation.

Overall, this experiment supports the efficiency of the SVD filter combined with SNR, and demonstrates the efficiency of the convolution neural network with a raw matrix as input and the efficiency of the convolution neural network with a filtered matrix as input for the identification of cavitation.

**Claims**

1. A computer-implemented method for identifying cavitation produced by an ultrasound therapy device, the ultrasound therapy device comprising at least one transducer configured for emitting ultrasound waves at a target and for receiving backscattered signal from the target, the method comprising the following steps of:

   a) arranging in a raw matrix a series of sampled acquired measurements signals, each sampled acquired measurement signals representing a backscattered signal received from the target by the at least one transducer subsequently to the emission by the at least one transducer of an emitting signal configured to nucleate cavitation inside the target, the sampled acquired measurements signals being aligned in the matrix to match their respective samples to the same time acquisition after the beginning of the emitting signal.

   b) determining from the matrix the presence or absence of one or more variable samples values through the series of sampled acquired measurements signals corresponding to cavitation.

2. The computer-implemented method according to claim 1, wherein step b) is at least partially carried out by an artificial intelligence engine.

3. The computer-implemented method according to 1 or 2, wherein the method comprises a preliminary acquisition step before step a) of carrying out a series of measures, each measure comprising acquiring the backscattered signal received from the target by the at least one transducer as a measurement signal.

4. The computer-implemented method according to claim 1 or 2, wherein step b) is carried out by:

   i) filtering the raw matrix by applying a filter configured for identifying and treating at least one sample corresponding to at least one echo of the emitting signal returned by the target by removing or lowering its value, and obtaining filtered samples, and

   ii) determining from the filtered matrix the presence of one or more samples of the raw matrix corresponding to cavitation.

5. The computer-implemented method according to claim 4, wherein substep ii) of step b) is carried out by an artificial intelligence engine having data structure which contains information reflecting relationship between values of the samples and the presence of cavitation.

6. The computer-implemented method according to claim 4 or 5, wherein the at least one echo of the emitting signal is a stable echo showing one or more stable sample value along the series of sampled acquired measurements signals and wherein the filter applied in substep i) of step b) is configured for identifying and removing stable samples along the series of sampled acquired measurements signals.

7. The computer-implemented method according to claim 6, wherein the filter is configured to separate stable sample values from unstable sample values along the series of sampled acquired measurements signals, preferably the filter carries out a singular value decomposition of the raw matrix.

8. The computer-implemented method according to any of claims 4, 6 and 7, wherein step b) comprises before substep i) the determination of an echo window comprising at least one echo of the emitting signal and devoid of cavitation, and wherein substep ii) of step b) comprises the determination for each filtered sampled signal of the filtered matrix of a signal-to-noise ratio indicator as the maximal value of the of the filtered samples divided by the standard deviation of the echo window, and wherein one or more samples of the raw matrix corresponding to cavitation is determined as present if the signal-to-noise ratio indicator is above a predetermined threshold.

9. The computer-implemented method according to any of claims 4, 6 and 7, wherein substep ii) of step b) comprises the determination of an amplitude indicator as the mean of the maximal filtered sample value of each filtered sampled signal of the filtered matrix, and wherein one or more samples of the raw matrix corresponding to cavitation is determined as present if the amplitude indicator is above a predetermined threshold.

10. The computer-implemented method according to any of claims 4, 6 and 7, wherein substep ii) of step b) comprises the determination for each filtered sampled signal of the filtered matrix of an energy indicator as the square root of the integral of the filtered samples values, and wherein one or more samples of the raw matrix corresponding to cavitation is determined as present if the energy indicator is above a predetermined threshold.

11. A computer system configured to communicate with an ultrasound therapy device, the ultrasound therapy device comprising: at least one transducer configured for emitting ultrasound waves at a target and for receiving backscattered signal from the target,
the computer system comprising one or more processors, a memory and one or more programs, wherein the one or more programs are stored in the memory and configured to be executed by the one or more processors, the one or more programs including instructions for performing, when executed by the computer system, a method for identifying cavitation according to any of claims 1 to 10.

12. The computer system according to claim 11, wherein the one or more programs includes instructions for performing a preliminary step upstream step a) of carrying out a series of measures, each measure comprising the following sub-steps:

   - emitting by the at least one transducer a signal configured to nucleate cavitation at the focal zone of the target, and
   - acquiring the echoes received from the target by the at least one transducer as a measurement signal.

13. A computer program product, stored on a non-transitory computer-readable data-storage medium comprising computer-executable instructions to cause a computer system to carry out a method for identifying cavitation according to any of claims 1 to 10.

14. A non-transitory computer-readable data-storage medium containing computer-executable instructions to cause a computer system to carry out a method for identifying cavitation according to any of claims 1 to 10.

15. An apparatus comprising the computer system according to claim 11 or 12 and an ultrasound therapy device configured for detection of cavitation, the ultrasound therapy device comprising: at least one transducer configured for emitting ultrasound waves at a target and for receiving backscattered signal from the target.

**Fig. 1**

# Fig. 2

```
┌─────────────────────────────────────────────┐
│ Acquisition of a series of backscattered     │
│ signals from a target in response to         │
│ ultrasound waves configured for nucleating   │
│ cavitation into the target                   │
└─────────────────────────────────────────────┘
                        │
                        ▼
        ┌───────────────────────────────┐
        │ Sampling the series of signals │
        └───────────────────────────────┘
                        │
                        ▼
        ┌───────────────────────────────┐
        │ Arranging the sampled signals  │
        │           into a matrix        │
        └───────────────────────────────┘
                  │                │
                  │                ▼
                  │         ┌──────────────┐
                  │         │   Filtering   │
                  │         │  the matrix   │
                  │         └──────────────┘
                  │                │
                  ▼                ▼
        ┌───────────────────────────────────┐
        │ Determining presence or absence of │
        │ sample(s) corresponding to         │
        │ cavitation                         │
        └───────────────────────────────────┘
```

**Fig. 3**

**Fig. 4**

**Fig. 5**

**Fig. 6**

**Fig. 7**

**Fig. 8**

**Fig. 9**

**Fig. 10**

**A**

**B**

**Fig. 11**

**Fig. 12**

## Fig. 13

**A**

**B**

## Fig. 14

## Fig. 15

## Fig. 16

**A**

**B**

## Fig. 17

**A**

**B**

**EUROPEAN SEARCH REPORT**

Application Number

EP 23 30 7374

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 801 761 B1 (CARDIAWAVE SA [FR]; INST NAT SANTE RECH MED [FR] ET AL.) 6 July 2022 (2022-07-06) * paragraphs [0008], [0022], [0025], [0026], [0034] – [0036], [0038], [0040] – [0048] * * figures 1,2,4 * | 1-15 | INV. A61N7/02 A61B8/08 ADD. A61N7/00 |
| A | US 2019/269385 A1 (EBBINI EMAD S [US] ET AL) 5 September 2019 (2019-09-05) * paragraph [0169] * * figures 8E,8 * | 1-15 | |
| A | SUAREZ ESCUDERO DANIEL ET AL: "2D and 3D real-time passive cavitation imaging of pulsed cavitation ultrasound therapy in moving tissues", PHYSICS IN MEDICINE & BIOLOGY, [Online] vol. 63, no. 23, 6 December 2018 (2018-12-06), page 235028, XP055844319, DOI: 10.1088/1361-6560/aaef68 Retrieved from the Internet: URL:http://iopscience.iop.org/article/10.1088/1361-6560/aaef68> [retrieved on 2024-05-13] * page 3 – page 6 * | 1-15 | |
| A | WO 2018/138576 A1 (INSIGHTEC LTD [IL]) 2 August 2018 (2018-08-02) * paragraphs [0043] – [0050] * * figures 7B,8B * | 1-15 | TECHNICAL FIELDS SEARCHED (IPC) A61N A61B |
| A | WO 2019/234497 A1 (INSIGHTEC LTD [IL]) 12 December 2019 (2019-12-12) * figures 5A,5B * | 1-15 | |

The present search report has been drawn up for all claims

| Place of search | Date of completion of the search | Examiner |
|---|---|---|
| The Hague | 13 May 2024 | Milles, Julien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 23 30 7374

This annex lists the patent family members relating to the patent documents cited in the above-mentioned European search report.
The members are as contained in the European Patent Office EDP file on
The European Patent Office is in no way liable for these particulars which are merely given for the purpose of information.

13-05-2024

| Patent document cited in search report | | | Publication date | Patent family member(s) | | Publication date |
|---|---|---|---|---|---|---|
| EP 3801761 | B1 | | 06-07-2022 | CA | 3101381 A1 | 28-11-2019 |
| | | | | CN | 112566694 A | 26-03-2021 |
| | | | | EP | 3801761 A1 | 14-04-2021 |
| | | | | FR | 3081334 A1 | 29-11-2019 |
| | | | | IL | 278882 A | 31-01-2021 |
| | | | | JP | 7358391 B2 | 10-10-2023 |
| | | | | JP | 2021523776 A | 09-09-2021 |
| | | | | KR | 20210027249 A | 10-03-2021 |
| | | | | US | 2021212709 A1 | 15-07-2021 |
| | | | | WO | 2019224350 A1 | 28-11-2019 |
| US 2019269385 | A1 | | 05-09-2019 | EP | 2579944 A2 | 17-04-2013 |
| | | | | EP | 3406299 A1 | 28-11-2018 |
| | | | | KR | 20130106345 A | 27-09-2013 |
| | | | | KR | 20180080357 A | 11-07-2018 |
| | | | | US | 2013144165 A1 | 06-06-2013 |
| | | | | US | 2019269385 A1 | 05-09-2019 |
| | | | | US | 2022031287 A1 | 03-02-2022 |
| | | | | US | 2023346354 A1 | 02-11-2023 |
| | | | | WO | 2011156624 A2 | 15-12-2011 |
| WO 2018138576 | A1 | | 02-08-2018 | CN | 110248606 A | 17-09-2019 |
| | | | | EP | 3573533 A1 | 04-12-2019 |
| | | | | JP | 7026118 B2 | 25-02-2022 |
| | | | | JP | 2020505134 A | 20-02-2020 |
| | | | | US | 2018206816 A1 | 26-07-2018 |
| | | | | WO | 2018138576 A1 | 02-08-2018 |
| WO 2019234497 | A1 | | 12-12-2019 | CN | 112236195 A | 15-01-2021 |
| | | | | EP | 3801763 A1 | 14-04-2021 |
| | | | | JP | 2022500093 A | 04-01-2022 |
| | | | | US | 2021204915 A1 | 08-07-2021 |
| | | | | WO | 2019234497 A1 | 12-12-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016156989 A

**Non-patent literature cited in the description**

- **S. I. MADANSHETTY ; R. A. ROY ; R. E. APFEL**. *J. Acoust. Soc. Am.*, September 1991, vol. 90 (3), 1515-1526
- **R. A. ROY ; S. I. MADANSHETTY ; R. E. APFEL**. *J. Acoust. Soc. Am*, June 1990, vol. 87 (6), 2451-2458
- **K.-A. SAALBACH ; J. TWIEFEL ; J. WAL-LASCHEK**. *Ultrasonics*, April 2019, vol. 94, 401-410
- **J. R. SUKOVICH et al.** *IEEE Trans. Ultrason. Ferroelectr. Freq. Control*, June 2020, vol. 67 (6), 1178-1191